# EUROPEAN PATENT APPLICATION

(11) **EP 4 621 052 A1**
(43) Date of publication of application: **24.09.2025**
(21) Application number: 23910074.6
(22) Date of filing: 11.12.2023
(51) Int. Cl.: C12N 9/12, C12N 15/54, C12N 15/11, A61K 49/00, A61K 47/64

(54) **IMMUNE CELL TARGETING PROBE, PRE-TARGETING RECOGNITION AND DRUG DELIVERY SYSTEM BASED ON PROBE, AND USE THEREOF**

(30) Priority: 29.12.2022 CN 202211728057
(71) Applicant: Guangdong Keyangle Life Technology Co., Ltd., Jiangmen, Guangdong 529000 (CN)
(72) Inventor: WEI, Yuan'an, Jiangmen, Guangdong 529000 (CN); LI, Chaowen, Jiangmen, Guangdong 529000 (CN)
(74) Representative: Clark, Claudia
(86) International application number: PCT/CN2023/137915
(87) International publication number: WO 2024/140144

(57) **Abstract**

Provided are an immune cell targeting probe, a pre-targeting recognition and drug delivery system based on the probe, and a use thereof. A targeting polypeptide and an antibody do not affect their own recognition effects after modification of a pre-targeting binding handle. The targeting polypeptide and the antibody can be self-assembled to form a combined probe, and the self-assembled probe can enhance the recognition capability to immune cells. Different targeting drug delivery strategies such as pre-targeting secondary delivery can be used on the basis of a difference in steric hindrance of binding of a probe to a target. Therefore, an off-target effect during delivery of toxic drugs is avoided or mitigated, and a use range of the toxic drugs is expanded. Due to the fact that a drug delivery ligand and a target recognition function portion can be independently subjected to chemical modification and storage, the drug delivery ligand can further load various drug molecules or modification groups, and has the advantages of high drug loading capacity, convenient synthesis, controllable product quality, etc., and has wide application in the aspects of immune cell imaging, tracing, screening, analysis and targeting drug delivery.

## Description

### TECHNICAL FIELD

The present disclosure belongs to the field of immunoassay, diagnosis and drug delivery, and in particular relates to an immune-cell-targeting probe and a pre-targeting recognition and drug delivery system based on the probe, and use thereof.

### BACKGROUND

Cell-targeting drug delivery systemsemploy drug carriers thatrecognizephysiological characteristics and biomarkers specific for target cell population to deliver drugs to the target cell area, which is an advanced and extremely complex and sophisticated drug delivery system. Compared with traditional drug delivery systems, cell-targeting drug delivery systems have unique advantages in terms of increased circulation timein *vivo,* targeted recognition, and biological compatibility, making them the optimal carriers for achieving multifunctional drug delivery and targeted therapy. However, the current cell-targeting drug delivery system is relatively in scope, mainly targeting erythrocytes and tumor cells, and often needs to be used in combination with other technologies, resulting in complex operations and high cost. For example, based on the hypotonic loading technology, dexamethasone sodium phosphate (Dex 21-P) is loaded into erythrocytes to treat ataxia telangiectasia. And based on the red blood cell-coated enzyme and antigen technology, L-asparaginase is loaded inside the red blood cells for the research of metastatic pancreatic cancer, metastatic breast cancer, and acute lymphatic leukemia.And based on the red blood cell-coated cytidine phosphorylase, hyperhomocysteinemia and related diseases are improved, etc (Li W, Su Z, Hao M, et al. Cytopharmaceuticals: An emerging paradigm for drug delivery[J]. Journal of Controlled Release, 2020, 328: 313-324.); the hemoglobin-poly (ε-caprolactone) (Hb-PCL) conjugate polymer-based self-assembled biomimetic nano-red blood cell delivery system (Wang Y, Yu J, Luo Z, et al. Engineering endogenous tumor-associated macrophage-targeted biomimetic nano-rbc to reprogram tumor immunosuppressive microenvironment for enhanced chemo-immunotherapy[J]. Advanced Materials, 2021, 33(39): 2103497.).And the ssDNA nanotube-based targeted therapy for glioblastoma (Harris M A, Kuang H, Schneiderman Z, et al. SsDNA nanotubes for selective targeting of glioblastoma and delivery of doxorubicin for enhanced survival[J]. Science advances, 2021, 7(49): eabl5872.).

The cell-targeting drug delivery systems is advantageous in treatment of diseases due to their prolonged circulation, active targeting, and ability to cross physiological barriers, etc.. However, the cell-targeting drug delivery system is still in the early stages of research. Currently, only red blood cells have entered the clinical trial stage as drug carriers, and several challenges remain to be addressed. For targeted drug delivery technology, off-target effects caused by systemic administration have always been an important issue that researchers need to overcome and avoid. These off-target effects can sometimes cause severe toxic side effects. Compared to the prolonged circulation and sustained-release drug delivery features provided by red blood cells, the immune cell-targeting drug delivery system employs immune cells as drug carriers and utilizes their natural tissue or organ tropism to selectively deliver drugs to specific lesion sites, increasing on-target effects and reducing off-target effects.

The central role of mammalian immune system is to guard the body from pathogens. The body's immune system includes innate immunity system and adaptive immunity system, with immune cells serving as the main executors of the immune system's functions. Cells within immune system can be further divided into lymphoid cells and myeloid-derived cells. Myeloid-derived cells include several major types of cells, including monocytes, neutrophils, dendritic cells, and tissue macrophages. The system composed of these cells is also called mononuclear phagocytic system (MPS). The MPS plays a key role in the initiation, progression, and resolution of inflammation. In addition, there are self-recognition signals on the surfaces of myeloid-derived cells possess and drugs coupled to them will have a longer residence time in the body. Therefore, the immune cell-mediated drug delivery system should in principle be able to effectively compensate for the defects of drug-loaded nanoparticles, such as the difficulty of drug-loaded nanoparticles to pass through endothelial cell layers in the body and the ease of drug-loaded nanoparticles being cleared by the MPS in the circulatory system. The immune cell-mediated drug delivery system can improve therapeutic efficacy of drugs, prolong the half-life of drugs, reduce immunogenicity and cytotoxicity, and, most notably, minimize off-target effects of drugs. As a result, the immune cell-mediated drug targeting delivery system has great development potential and has gradually become a research and development hotspot.

Radioimmunotherapy (RIT) is a "targeted" diagnostic and radiotherapy method developed based on monoclonal antibody technology in the late 20th century. It can specifically kill tumor cells by coupling radioactive isotopes onto monoclonal antibodies targeting specific tumor cells, without harming the surrounding healthy tissues of the lesion. However, the existing RIT technology generally face a challenge that the dose of radioactive drugs delivered to tumors (i.e., the ratio of targeted to non-targeted radioactivity (T/NT)) is not insufficient (i.e., the off target effect is too high). The main reason is that the current monoclonal antibodies used to couple radionuclides have an excessively long half-life in bloodstream (an average half-life is 7-21 days, depending on the type of isotopes). For the toxicity of radioactive isotopes, the excessively long half-life allows them to circulate in the body for sufficient time and induce damage to the hematopoietic system, causing great harm to the body and limiting the practical application of this technology. So, based on this, the pretargeted radioimmunotherapy (PRIT) technology has been developed. The basic principle of PRIT is to administer the targeting antibody molecule separately from the radionuclide. First, a targeting agent (antibody) is administered to fully bind to a specific target. This step is usually performed several hours to several days before the administration of a radioactive component or drug, so that these targeting agents (antibodies) have sufficient time to accumulate on the target. Once the targeting agent has optimally localized and concentrated at the target, a radioactive ligand or drug delivery ligand (i.e., a radionuclide-labeled effector molecule or a specific drug carrier) that can specifically recognize the pre-targeted targeting agent is administered, thereby delaying the administration of the radioactive ligand or toxic drug. The delayed administration time can be from several hours to several days, thereby improving the rapid and efficient targeting of the radionuclide-labeled ligand molecule or drug carrier to the specific target in the body, and at the same time greatly reducing the metabolic retention time of off-target radionuclide-labeled ligand molecules or drug carriers in the body, and improving the clearance rate and reducing off-target toxicity.

### SUMMARY

In order to achieve the above-mentioned objectives, an immune-cell-targeting probe and a pre-targeting recognition and drug delivery system based on the probe and use thereof are proposed in present disclosure. In the present disclosure, the immune-cell-targeting probe is a novel immune cell targeting probe constructed by pre-targeting modification of the targeting polypeptides, antibodies, and combination probes(connecting pre-targeting binding handles). The combination probe is formed by self-assembly of polypeptides (targeting polypeptides) previously discovered by the inventors that can target immune cells and antibodies prepared from these polypeptides as immune antigens, or targeting polypeptide and its antibody. In addition, based on the pre-targeting binding handle installed, the drug delivery ligand with a linker can be delivered to the target cells efficiently and accurately. Moreover, usingdifferent specific drug delivery strategies, non-orless-totoxicities directional delivery of cytotoxic substances can be achieved, reducing the circulation time of cytotoxic substances in the body, broadening the selectivity and application of cytotoxic substances, and improving their safety of use.

In a first aspect of the present disclosure, an immune-cell-targeting probe is provided, including the following (1), or a combination of (1) and (2):
(1) a targeting polypeptide connected with a pre-targeting moiety (refer as binding handle thereafter); and
(2) an antibody or a functional fragment thereof prepared from the targeting polypeptidein (1) as an immunogen, and connected with the pre-targeting binding handle.

In some embodiments of the present disclosure, the immune-cell-targeting probe is a combination probe formed by combining the targeting polypeptide and the antibody or the functional fragment thereof prepared from the targeting polypeptide as an immune antigen, that is, when (1) and (2) are used in combination.

In some embodiments of the present disclosure, the inventors have found that using the combination probe has better targeting effects, stronger targeting binding affinity and more excellent flow cytometry screening and analysis effects compared to using a relatively single targeting polypeptide or antibody prepared from the targeting polypeptide as the immune antigen.

In some embodiments of the present disclosure, when (1) and (2) are used in combination, the probe contains one or more pre-targeting binding handles, and the pre-targeting binding handles may be present respectively or individually on at least one of the targeting polypeptide and/or the antibody, or the functional fragment thereof.

In some embodiments of the present disclosure, without affecting the recognition efficiency of the targeting polypeptide, the antibody or the functional fragment thereof, and the combination probe, the pre-targeting binding handle may be modified and connected to a specific position of the three, and one or more handles may be connected according to actual use needs.

In some embodiments of the present disclosure, when (1) and (2) are used in combination, the probe contains 1 to 2 pre-targeting binding handles.

In some embodiments of the present disclosure, when (1) and (2) are used in combination, the probe contains one pre-targeting binding handle.

In some embodiments of the present disclosure, the pre-targeting binding handles are respectively present on the targeting polypeptide and the antibody or functional fragment thereof.

In some embodiments of the present disclosure, the pre-targeting binding handle is individually present on the antibody or the functional fragment thereof.

In some embodiments of the present disclosure, the probe is further connected to at least one of a linker and a marker.

In some embodiments of the present disclosure, the probe may be connected to the marker via the linker, or directly connected to the marker.

In some embodiments of the present disclosure, the marker includes a reporter group and a carrier.

In some embodiments of the present disclosure, the reporter group is a chromogenic enzyme, a fluorescent labeling group, a chemiluminescent labeling group, an isotope or a magnetic functional group.

In some embodiments of the present disclosure, the chromogenic enzyme includes peroxidase or alkaline phosphatase.

In some embodiments of the present disclosure, the fluorescent labeling group includes fluorescent proteins, rhodamines, fluoresceins, cyanine dyes, Alexa Fluor dyes and/or quantum dot fluorescent groups.

In some embodiments of the present disclosure, the magnetic functional group includes groups capable of magnetic resonance imaging and changing relaxation efficiency.

In some embodiments of the present disclosure, the isotope includes a radionuclide.

In some embodiments of the present disclosure, the radionuclide includes radionuclides used in radioimmunoimaging and radioimmunotherapy.

In some embodiments of the present disclosure, the pre-targeting binding handle specifically recognizes and binds to the linker.

In some embodiments of the present disclosure, a binding mode of the pre-targeting binding handle and the linker includes highly specific recognition systems such as biotin-avidin, complementary nucleic acid molecules, and antigen-antibody.

In a specific embodiment of the present disclosure, the inventors mainly use the biotin-avidin system as a stable specific recognition means. For the application of this means in targeted therapy, please refer to Lesch HP, Kaikkonen MU, Pikkarainen JT, et al. Avidin-biotin technology in targeted therapy[J]. Expert Opinion on Drug Delivery, 2010, 7(5): 551-564.

In some embodiments of the present disclosure, the targeting polypeptide is a C-terminal fragment sequence of Triokinase/FMN cyclase, including:
(a) an amino acid sequence containing VLQ;
(b) an amino acid sequence having more than 80% identity with the amino acid sequence VLQ described in (a), and the amino acid sequence is of non-human animal origin; and
(c) a variant of the amino acid sequence described in (a) or (b).

In a specific embodiment of the present disclosure, regarding the species origin of the Triokinase/FMN cyclase and the "animals" mentioned above, there is no particular limitation on the types of animals, for example, including birds and mammals. Examples of birds include poultry such as chickens and ducks; examples of mammals include mice, rats, rabbits, pigs, dogs, cows, primates (e.g., humans), and the like. Herein, non-human animals specifically refer to mammals excluding humans and birds. In other words, the polypeptide sequence at the C-terminus of the Triokinase/FMN cyclase is a relatively conservative sequence at least in mammals and birds, and mutations of amino acid residues at certain sites do not affect its function of targeting immune cells.

In some embodiments of the present disclosure, the amino acid sequence described in (b) has more than 80%, 85%, 90% or 95% identity with the amino acid sequence described in (a).

In some embodiments of the present disclosure, the amino acid sequence described in (b) has more than 80%, 81%, 82%, 83%, 84%, 85%, 86%, 87%, 88%, 89%, 90%, 91%, 92%, 93%, 94%, 95%, 96%, 97%, 98% or 99% identity with the amino acid sequence described in (a).

In some embodiments of the present disclosure, the amino acid sequence described in (b) may be obtained by modifying or substituting one or more amino acids in the amino acid sequence described in (a). The specific method for such modification and substitution can use any method known in the prior art.

In some embodiments of the present disclosure, the amino acid sequences described in (a), (b) and (c) include 45 or fewer amino acid residues. They may include 45, 44, 43, 42, or 41 amino acid residues. In some embodiments of the present disclosure, the amino acid sequences described in (a) and (b) include 41 or fewer amino acid residues.

In some embodiments of the present disclosure, the amino acid sequences described in (a), (b) and (c) include 9 to 41 amino acid residues.

In some embodiments of the present disclosure, the C-terminal fragment sequence of the Triokinase/FMN cyclase described in (a) includes an amino acid sequence that has more than 80% identity with the amino acid sequence as shown in SEQ ID NOs: 2 to18 and 29.

In some embodiments of the present disclosure, when (1) and (2) are used in combination, the targeting polypeptide contains VLQ in the C-terminal fragment sequence of Triokinase/FMN cyclase. When (1) is used alone, the targeting polypeptide is not limited to the C-terminal fragment sequence of Triokinase/FMN cyclase and may not contain VLQ.

In some embodiments of the present disclosure, the C-terminal fragment sequence of Triokinase/FMN cyclase is used to prepare the immune antigen of the above-mentioned antibody, and the C-terminal fragment sequence of Triokinase/FMN cyclase is:
(d) LDQPDPGAVAAAAIFRAILEVLQTQGA (SEQ ID NO: 7) or a variant thereof;
wherein the variant includes an amino acid sequence having more than 80% identity with the amino acid sequence described in (d), and the variant contains VLQ.

In some embodiments of the present disclosure, the amino acid sequence of the variant described in (d) has more than 80%, 85%, 90% or 95% identity with the sequence shown in (c).

In some embodiments of the present disclosure, the amino acid sequence of the variant in (d) has more than 80%, 81%, 82%, 83%, 84%, 85%, 86%, 87%, 88%, 89%, 90%, 91%, 92%, 93%, 94%, 95%, 96%, 97%, 98% or 99% identity with the sequence shown in (c).

In some embodiments of the invention, an antibody KA-001 is obtained based on the sequence LDQPDPGAVAAAAIFRAILEVLQTQGA (SEQ ID NO:7).

In some embodiments of the invention, the variant in (d) has a sequence shown in EQPDPGAVAAAAILRAILEVLQS (SEQ ID NO: 12). An antibody KA-004 is obtained based on this variant.

In some embodiments of the present disclosure, the amino acid sequence described in (d) includes 45 or fewer amino acid residues.

In some embodiments of the present disclosure, the amino acid sequence described in (d) includes 41 or fewer amino acid residues. The amino acid sequence described in (d) includes 9 to 41 amino acid residues, and may include 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or 41 amino acid residues.

In the present disclosure, the term "Triokinase/FMN cyclase" refers to an enzyme that catalyzes phosphorylation of dihydroxyacetone and glyceraldehyde, and cleavage of ribonucleoside diphosphate-X compounds, of which FAD is the optimal substrate. Triokinase/FMN cyclase (also known as DAK protein) is first discovered in prokaryotic/eukaryotic microorganisms (see Erni B, Siebold C, Christen S, et al. Small substrate, big surprise: Fold, function and phylogeny of dihydroxyacetone kinases[J]. Cellular and Molecular Life Sciences, 2006, 63(7-8): 890-900.), and is reported to catalyze the phosphorylation of dihydroxyacetone to produce dihydroxyacetone phosphate (Dha-P). In 2005, it is found that the FAD-AMP lyase (also known as FMN cyclase) identified in rat liver extracts has homology with the amino acid sequence of microbial DAK protein, and it is confirmed that the DAK protein has dual functional properties of both kinase and cyclase. In 2007, it is reported that the DAK protein can inhibit RNA helicase MDA5-mediated cellular antiviral signaling (see Diao F, Li S, Tian Y, et al. Negative regulation of MDA5- but not RIG-I-mediated innate antiviral signaling by the dihydroxyacetone kinase[J]. Proceedings of the National Academy of Sciences, 2007, 104(28): 11706-11711.).

In addition, according to a specific embodiment of the present disclosure, the selection of the polypeptide corresponding to the C-terminal fragment of Triokinase/FMN cyclase (i.e., the polypeptide targeting and recognizing immune cells) in the present disclosure is mainly based on the inventor's prior Chinese patents CN 110885805 A, CN 111051504 A and CN 110760491 A, including but not limited to the sequences shown in Table 1 of the description thereof.

It should be noted that the present disclosure does not involve the protection of the polypeptide corresponding to the C-terminal fragment of Triokinase/FMN cyclase (i.e., a polypeptide targeting and recognizing immune cells), which is only introduced into the present disclosure as one of the necessary materials for preparing the antibody and probe described herein.

In some embodiments of the present disclosure, the antibody or the functional fragment thereof is an antibody prepared from the polypeptide corresponding to the C-terminal fragment of Triokinase/FMN cyclase (i.e., the targeting polypeptide) or the variant thereof as an immune antigen. The functional fragment refers to a truncated fragment that can realize the functionality of the antibody. Furthermore, the functional fragment refers to a truncated fragment of an antibody prepared by genetic engineering or antibody engineering that can realize the function of the antibody.

In some embodiments of the present disclosure, the targeting polypeptide further includes haptens and artificial antigens constructed based on the targeting polypeptide.

In some embodiments of the present disclosure, the artificial antigen includes the above-mentioned targeting polypeptide coupled to a protein carrier via a linker sequence amino acid (such as cysteine, Cys).

In some embodiments of the present disclosure, the protein carrier includes hemocyanin (KLH), serum albumin (ALB), and ovalbumin (OVA).

In some embodiments of the present disclosure, the serum albumin includes bovine serum albumin (BSA), human serum albumin (HSA), rabbit serum albumin (RSA), equine serum albumin (ESA) and goat serum albumin (GSA).

In some embodiments of the present disclosure, the protein carrier is coupled to the side-chain thiol of the linker sequence amino acid Cys which is artificially designed and added to the C-terminal fragment sequence of Triokinase/FMN cyclase.

In some embodiments of the present disclosure, the inventors have found that variants obtained by modifying the antigens, such as haptens or artificial antigens, can also have the ability to target immune cells when used as immunogens to generate antibodies by conventional immunization methods.

In a specific embodiment of the present disclosure, the results of ELISA antigen epitope detection show that the antibodies KA-001 and KA-004 of the present disclosure can specifically recognize the epitope composed of the VLQ sequence at the C-terminus of Triokinase/FMN cyclase.

In a specific embodiment of the present disclosure, the results of ELISA antigen epitope detection show that the antibodies KA-001 and KA-004 of the present disclosure can specifically recognize the epitope composed of the VLQS or VL sequence at the C-terminus of Triokinase/FMN cyclase.

In some embodiments of the present disclosure, the antibodies are the monoclonal antibodies prepared by conventional hybridoma techniques and screened.

Of course, it should be understood that the antibody can be prepared according to conventional antibody preparation methods in the art, including but not limited to genetic engineering, hybridoma technique and the like.

In some embodiments of the present disclosure, the antibody is a chimeric antibody, a humanized antibody or a fully human antibody.

In some embodiments of the invention, the variant is a truncated fragment capable of achieving the functionality of the antibody.

In some embodiments of the present disclosure, amino acid sequences of complementarity-determining regions (CDRs) of a heavy chain variable region of the antibody or the functional fragment thereof are shown by the amino acid sequences at positions 26 to 35, 50 to 66, and 99 to 110 of SEQ ID NO: 19, respectively; and amino acid sequences of the CDRs of a light chain variable region are shown by the amino acid sequences at positions 24 to 34, 50 to 56, and 89 to 97 of SEQ ID NO: 21, respectively.

In some embodiments of the present disclosure, the heavy chain variable region of the antibody or the functional fragment thereof is as shown in SEQ ID NO: 19; and the light chain variable region is as shown in SEQ ID NO: 21.

In some embodiments of the present disclosure, the antibody further includes a constant region.

In some embodiments of the present disclosure, the constant region is a humanized constant region.

In some embodiments of the present disclosure, the antibody is prepared from at least one amino acid sequence having at least 95% identity with the amino acid sequences shown in SEQ ID NOs: 1 to 18 as an immune antigen.

In some embodiments of the present disclosure, the amino acid sequence of the antigen has more than 95%, 96%, 97%, 98% or 99% identity with the amino acid sequences shown in SEQ ID NOs: 1 to 18.

The selection criteria for amino acid sequences having at least 95% identity with the amino acid sequences shown in SEQ ID NOs: 1 to 18 can refer to the inventor's prior Chinese patents CN 110885805 A, CN 111051504 A and CN 110760491 A, which include but are not limited to the sequences shown in Table 1 of the description of the present disclosure.

In some embodiments of the present disclosure, when (1) and (2) are used in combination, the antibody is additionally modified or connected with a small molecule active substance.

In some embodiments of the present disclosure, the small molecule active substance includes a polypeptide linker sequence, a biotin-avidin system, a chromogenic enzyme, a fluorescent labeling group, a chemiluminescent labeling group, an isotope or a magnetic functional group.

In some embodiments of the present disclosure, the linker sequence includes lysine (K), and cysteine (C);
the chromogenic enzyme includes peroxidase or alkaline phosphatase;
the fluorescent labeling group includes fluorescent proteins, rhodamines, fluoresceins, cyanine dyes, Alexa Fluor dyes and/or quantum dot fluorescent groups;
the magnetic functional groups include groups capable of magnetic resonance imaging and changing relaxation efficiency; and
the isotopes include radionuclides.

In some embodiments of the present disclosure, the radionuclide includes radionuclides used in radioimmunoimaging and radioimmunotherapy.

In some embodiments of the present disclosure, the antibodies target and recognize immune cells.

In some embodiments of the present disclosure, the immune cells include, but are not limited to, lymphocytes, dendritic cells, monocyte precursors, monocytes/macrophages, neutrophils, basophils, eosinophils, and mast cells.

In some embodiments of the present disclosure, the lymphocytes include B lymphocytes.

In some embodiments of the present disclosure, the lymphocytes include CD3⁺ T cells and CD5⁺ B cells.

In the present disclosure, there is no particular limitation on the source of immune cells, which include, for example, birds and mammals. Examples of birds include poultry such as chickens and ducks; and examples of mammals include mice, rats, rabbits, pigs, dogs, cows, primates (e.g., humans), and the like.

In second aspect of the present disclosure, the use of the probe described in the first aspect of the present disclosure in immune cell imaging, tracing, screening and analysis is provided.

In some embodiments of the present disclosure, the immune-cell-targeting probe can achieve different purposes of use such as drug delivery, imaging, tracing, screening and analysis based on the specific selection of the delivery substance. When the delivery substance is a reporter group, a non-drug carrier, etc., it can achieve imaging, tracing, screening and analysis effects based on the specific selection of the reporter group, non-drug carrier and other substances. In particular, when the delivery substance is a reporter group, probes labeled with different fluorescent colors can be obtained by mounting different reporter groups, providing a more convenient and flexible fluorescent color combination in flow cytometry analysis.

In some embodiments of the present disclosure, a method for using the immune-cell-targeting probe in immune cell imaging includes the steps of:
administering first to a subject or target a targeting polypeptide connected with a pre-targeting binding handle, followed by administering a drug delivery ligand; or
mixing the targeting polypeptide connected with the pre-targeting binding handle with the drug delivery ligand to obtain a mixture, followed by administering the mixture to the subject or target.

In some embodiments of the present disclosure, a method for using the immune-cell-targeting probe in immune cell imaging includes the steps of:
administering first a subject or target an antibody or a functional fragment thereof prepared from a targeting polypeptide connected with a pre-targeting binding handle as an antigen, followed by administering a drug delivery ligand; or
mixing the antibody or the functional fragment thereof prepared from the targeting polypeptide connected with the pre-targeting binding handle as the immunogen with the drug delivery ligand to obtain a mixture, followed by administering the mixture to the subject or target.

In some embodiments of the present disclosure, a method for using the immune-cell-targeting probe in immune cell imaging includes the steps of:
administering first to a subject or target a targeting polypeptide connected with a pre-targeting binding handle and an antibody or a functional fragment thereof prepared from the targeting polypeptide connected with the pre-targeting binding handle as an immune antigen, followed by administering a drug delivery ligand; or
mixing the targeting polypeptide connected with the pre-targeting binding handle, the antibody or the functional fragment thereof prepared from the targeting polypeptide connected with the pre-targeting binding handle as the immunogen with the drug delivery ligand to obtain a mixture, followed by administering the mixture to the subject or target.

In some embodiments of the invention, the imaging includes: imaging of *ex vivo* samples and direct *in vivo* drug delivery imaging.

In some embodiments of the present disclosure, the immune cells include, but are not limited to, lymphocytes, dendritic cells, monocyte precursors, monocytes/macrophages, neutrophils, basophils, eosinophils, and mast cells.

In some embodiments of the present disclosure, the lymphocytes include B lymphocytes.

In some embodiments of the present disclosure, the probe is a targeting polypeptide connected with a pre-targeting binding handle.

In some embodiments of the present disclosure, the targeting polypeptide is connected with one or more pre-targeting binding handles.

In some embodiments of the present disclosure, the targeting polypeptide is connected with 1 to 2 pre-targeting binding handles.

In some embodiments of the present disclosure, the targeting polypeptide is connected with one pre-targeting binding handle.

In some embodiments of the present disclosure, the probe is further connected with a linker and/or a marker.

In some embodiments of the present disclosure, the marker includes a reporter group and a carrier.

In some embodiments of the present disclosure, the reporter group is a chromogenic enzyme, a fluorescent labeling group, a chemiluminescent labeling group, an isotope or a magnetic functional group.

In some embodiments of the present disclosure, the chromogenic enzyme includes peroxidase and alkaline phosphatase.

In some embodiments of the present disclosure, the fluorescent labeling group includes fluorescent proteins, rhodamines, fluoresceins, cyanine dyes, Alexa Fluor dyes and/or quantum dot fluorescent groups.

In some embodiments of the present disclosure, the magnetic functional groups include groups capable of magnetic resonance imaging and changing relaxation efficiency.

In some embodiments of the present disclosure, the isotope includes a radionuclide.

In some embodiments of the present disclosure, the radionuclide includes radionuclides used in radioimmunoimaging and radioimmunotherapy.

In some embodiments of the present disclosure, the targeting polypeptide is shown by a sequence corresponding to the C-terminal fragment sequence of Triokinase/FMN cyclase, including: an amino acid sequence having more than 80% identity with the amino acid sequences shown in SEQ ID NOs: 1 to 18, 29.

In some embodiments of the present disclosure, the targeting polypeptide corresponding to the C-terminal fragment sequence of Triokinase/FMN cyclase may not contain VLQ.

In some embodiments of the invention, the targeting polypeptide includes 45 or less amino acid residues.

In some embodiments of the present disclosure, the amino acid sequence in the targeting polypeptide comprises 41 or fewer amino acid residues. The amino acid sequence in (d) includes 9 to 41 amino acid residues, and may include 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, or 41 amino acid residues.

In addition, according to a specific embodiment of the present disclosure, the selection of the polypeptide corresponding to the C-terminal fragment of Triokinase/FMN cyclase (i.e., the polypeptide targeting and recognizing immune cells) in the present disclosure is mainly based on the inventor's prior Chinese patents CN 110885805 A, CN 111051504 A and CN 110760491 A, including but not limited to the sequences shown in Table 1 of the description thereof.

In third aspect of the present disclosure, a detection product including the probe described in the first aspect of the present disclosure is provided. The detection product includes but is not limited to a detection reagent, a detection kit, and a detection chip.

In some embodiments of the present disclosure, the type of the detection product can be adjusted according to actual use requirements, and the preparation method thereof can be achieved according to conventional methods in the art. Exemplarily, when the probe described in the first aspect of the present disclosure is configured into a solution formulation, a detection reagent is obtained. After the detection reagent and other auxiliary reagents or products are assembled into the same kit, a detection kit is obtained. A detection chip is obtained by coating the probe described in the first aspect of the present disclosure in a microarray chip.

In fourth aspect of the present disclosure, an immune-cell-targeting drug delivery system including the probe described in the first aspect of the present disclosure and a delivery ligand is provided.

In some embodiments of the present disclosure, the probe is a combination probe formed by combining a targeting polypeptide and an antibody or a functional fragment thereof prepared from the targeting polypeptide as an immune antigen.

In some embodiments of the present disclosure, the delivery ligand includes a linker and a delivery drug.

In some embodiments of the present disclosure, the delivery ligand is a delivery drug connected with a linker.

In the present disclosure, the term "drug" refers to a substance used to prevent, treat and diagnose a disease, including chemical substances that can affect physiological functions of body organs and cell metabolic activities, all of which fall within the scope of the drug in the present disclosure.

In the present disclosure, the immune-cell-targeting drug delivery system can achieve immune-cell-targeting drug delivery based on a variety of different targeting strategies.

In some embodiments of the present disclosure, the targeting strategy of the immune-cell-targeting drug delivery system is as follows: the immune cells are pre-targeted and recognized using a targeting polypeptide connected with a pre-targeting binding handle in the immune-cell-targeting drug delivery system, and then a drug delivery ligand (i.e., the above-mentioned delivery ligand) is added to recognize and bind to the pre-targeting binding handle on the targeting polypeptide that has been fixed on the immune cells based on the linker on the drug delivery ligand, thereby delivering the drug to the immune cells in a targeted manner (pre-targeting drug delivery strategy).

In some embodiments of the present disclosure, the targeting strategy of the immune-cell-targeting drug delivery system is as follows: the targeting polypeptide and the antibody or the functional fragment thereof are pre-mixed *in vitro* and then injected into the body, or injected separately and allowed to self-assemble *in vivo.* The targeting polypeptide and the antibody or the functional fragment thereof form a combination probe for pre-targeted recognition of immune cells, and then the drug delivery ligand is added. The linker on the drug delivery ligand recognizes and binds to the pre-targeting binding handle in the combination probe that has been fixed on the immune cells, thereby delivering drugs to the immune cells in a targeted manner (enhanced pre-targeting drug delivery strategy).

In the present disclosure, the term "pre-targeting" is derived from the pretargeted radioimmunotherapy (PRIT) technology. The basic principle of the PRIT is to administer the targeting antibody molecule separately from the radionuclide. In the present disclosure, the main points of pre-targeting are as follows: first, the targeting substance (in the present disclosure, the targeting polypeptide, the antibody or the functional fragment thereof, or the combination probe formed by their self-assembly) is administered to fully bind to the target. This step is usually performed several hours to several days before the administration of the radioactive component or drug, so that these targeting substances have time to fully accumulate on the target. When the positioning and concentration of the targeting substance on the target are optimized, a radioactive ligand or drug delivery ligand (a radionuclide-labeled effector molecule or a specific drug carrier) that can specifically recognize the pre-targeted targeting substance is administered, thereby delaying the administration of the radioactive ligand or drug. The delayed administration time can be from several hours to several days, thereby improving the rapid and efficient targeting of the radionuclide-labeled ligand molecule or drug carrier to the specific target *in vivo,* and at the same time greatly reducing the metabolic retention time of the off-target radioactive nuclide-labeled ligand molecule or drug carrier in the body, that is, improving the clearance rate and reducing off-target toxicity.

In the related technologies, although the pre-targeting technology has certain preclinical and clinical research results in RIT and it has been confirmed that the pre-targeting technology can indeed increase the ratio of tumor to non-tumor radioactivity, there have been no related reports indicating its application in drug delivery, especially the related technologies have not disclosed its targeted drug delivery system targeting immune cells.

The targeting polypeptides disclosed in the inventor's prior Chinese granted patents CN 111051504 A and CN 110760491 A, when carrying imaging reporter groups, can perform *in situ* imaging of various monocytes/macrophages, clearly showing the *in vivo* distribution and spatiotemporal changes of the relevant monocytes/macrophages, thereby studying the physiological functions and real-time distribution of monocytes/macrophages *in vivo.* In the present disclosure, based on the targeting polypeptide, the inventors have further developed a biotin-modified version combined with avidin of such polypeptides, antibodies prepared from such polypeptide as an immune antigen, and a combination probe formed by their self-assembly, and discovered their use as a novel cell targeting probe. This application effectively overcomes the defects of traditional cell-targeting vectors and can perform pre-targeting functions *in vivo.* Not only does it realize the *in situ* imaging function of the original targeting polypeptide, but it also finds that the combination probe according to the present disclosure has stronger imaging, tracing, screening and analysis effects than the original targeting polypeptide.

In some embodiments of the present disclosure, the immune cells include, but are not limited to, lymphocytes, dendritic cells, monocyte precursors, monocytes/macrophages, neutrophils, basophils, eosinophils, and mast cells.

In some embodiments of the present disclosure, the lymphocytes include B lymphocytes.

In some embodiments of the present disclosure, the lymphocytes include CD3⁺ T cells and CD5⁺ B cells.

In fifth aspect of the present disclosure, an immune-cell-targeting drug delivery method is provided, comprising the following steps:
administering first to a subject or target a targeting polypeptide connected with a pre-targeting binding handle, followed by administering a drug delivery ligand (pre-targeting drug delivery strategy); or
mixing the targeting polypeptide connected with the pre-targeting binding handle with the drug delivery ligand to form a mixture, followed by administering the mixture to the subject or target (one-step self-assembly drug delivery strategy).

In sixth aspect of the present disclosure, an immune-cell-targeting drug delivery method is provided, comprising the following steps:
administering first to a subject or target an antibody or a functional fragment thereof prepared from a targeting polypeptide connected with a pre-targeting binding handle as an immune antigen, followed by administering a drug delivery ligand (pre-targeting drug delivery strategy); or
mixing the antibody or the functional fragment thereof prepared from the targeting polypeptide connected with the pre-targeting binding handle as the immune antigen with the drug delivery ligand to form a mixture, followed by administering the mixture to the subject or target (one-step self-assembly drug delivery strategy).

In seventh aspect of the present disclosure, an immune-cell-targeting drug delivery method is provided, comprising the following steps:
administering first to a subject or target a targeting polypeptide connected with a pre-targeting binding handle and an antibody or a functional fragment thereof prepared from the targeting polypeptide connected with a pre-targeting binding handle as an immune antigen, followed by administering a drug delivery ligand (pre-targeting drug delivery strategy); or
mixing the targeting polypeptide connected with the pre-targeting binding handle and the antibody or the functional fragment thereof prepared from the targeting polypeptide connected with the pre-targeting binding handle as the immune antigen with the drug delivery ligand to form a mixture, followed by administering to the subject or the target (one-step self-assembly drug delivery strategy).

According to the fifth, sixth, and seventh aspects of the present disclosure, in some embodiments, the administration includes intraperitoneal injection, pulmonary inhalation, direct *in vitro* administration.

In some embodiments of the present disclosure, the administration mode is determined based on the subject and environment of use. In some embodiments of the present disclosure, when the subject to be used is an *in vivo* environment, the administration includes intraperitoneal injection and pulmonary inhalation. When the subject to be used is an *in vitro* or *ex vivo* cell, tissue or the like, the administration includes direct administration to the subject.

In some embodiments of the present disclosure, when the method involves multiple administrations, the administration modes are the same or different.

In some embodiments of the present disclosure, when the method involves multiple administrations, the administration modes are the same, such as intraperitoneal injection.

In some embodiments of the present disclosure, the pre-targeting binding handle can be connected to either the targeting polypeptide or the antibody alone, or the pre-targeting binding handle can be connected to both the targeting polypeptide or the antibody. The number of pre-targeting binding handles can be adjusted according to actual usage requirements.

In some embodiments of the present disclosure, the pre-targeting binding handle is only connected to the antibody, with one handle per antibody. Of course, it should be understood that this example should not be construed as limiting the combination probe of the present disclosure.

In eighth aspect of the present disclosure, use of the immune-cell-targeting drug delivery system according to the fourth aspect of the present disclosure in drug delivery is provided.

In ninth aspect of the present disclosure, the use of the immune-cell-targeting drug delivery system according to the fourth aspect of the present disclosure in immune cell recognition and pre-targeting is provided.

In some embodiments of the present disclosure, the immune-cell-targeting drug delivery system according to the fourth aspect of the present disclosure can further utilize its immune cell recognition and pre-targeting functions to play a role in disease diagnosis and treatment, thereby achieving effective application in disease diagnosis and treatment.

In the present disclosure, based on the above-mentioned targeting polypeptides, the inventors have further developed a biotin-modified version combined with avidin of such polypeptides, antibodies prepared from the polypeptides as antigens, and a combination probe formed by their self-assembly, and discovered their applications in drug delivery. This application achieves high-drug loading and high-precision targeted drug delivery to immune cells. Based on its pre-targeting strategy, it can quickly and efficiently target immune cells *in vivo,* while greatly reducing the metabolic retention time of off-target reporter groups or drug molecules in the body, thereby improving clearance rate and reducing off-target toxicity.

The beneficial effects of the present disclosure are as follows:
1.The immune-cell-targeting drug delivery system of the present disclosure can achieve immune-cell-targeting drug delivery based on a targeting polypeptide connected with a pre-targeting binding handle, an antibody prepared from the targeting polypeptide connected with the pre-targeting binding handle as an immune antigen, or a combination probe formed by their self-assembly. Moreover, based on the specific selection of the carrier material, it can also be used for the marking and identification of immune cells, and has extremely high application value.
2. In the present disclosure, the combination probe obtained by self-assembly of the targeting polypeptide and the antibody prepared from the targeting polypeptide as the immune antigen has better recognition efficiency and recognition effect than the use of the targeting polypeptide or the antibody prepared from the targeting polypeptide as the immune antigen alone, and the assembly process is mild, and can be quickly assembled under appropriate conditions *in vivo* and *in vitro* without affecting its own recognition effect.
3. The immune-cell-targeting drug delivery system of the present disclosure can deliver drugs based on different drug delivery strategies, and produce different recognition effects based on different drug delivery strategies, such as one-time recognition and pre-targeted recognition, so as to meet the usage requirements of different delivery substances and effectively improve the recognition accuracy.
4. The immune-cell-targeting drug delivery system and probe of the present disclosure can achieve different usage purposes according to the needs, including drug delivery centered on self-assembly, and recognition, screening and analysis purposes by carrying other modified groups. The system or probe has a high carrying capacity, offers advantages such as ease of synthesis and controllable product quality, and can be widely used in conventional immune cell analysis and delivery of cytotoxic drugs and substances.

### BRIEF DESCRIPTION OF DRAWINGS

FIG. 1 shows a plasmid map of pcDNA3.4 according to an embodiment of the present disclosure, where A is the plasmid map of the heavy chain and B is the plasmid map of the light chain.
FIG. 2 shows the specific binding effect of KA-001 and KA-004 on VLQ.
FIG. 3 shows a schematic diagram of recognition modes of an immune cell-targeting probe according to the present disclosure, where A represents the first mode, B represents the second mode, and C represents the third mode.
FIG.4 shows a flow cytometric analysis diagram of a targeting marker using an antibody of the targeting polypeptide as an immune-cell-targeting probe.
FIG.5 shows a flow cytometric analysis diagram of the targeting marker of peritoneal macrophages *in vivo* by the targeting polypeptide connected with a pre-targeting binding handle.
FIG.6 shows a flow cytometric analysis diagram of the targeting marker of peripheral blood labeled with the targeting polypeptide connected with the pre-targeting binding handle.
FIG.7 shows a flow cytometric analysis diagram of CD5⁺ B lymphocytes labeled with the targeting polypeptide connected with the pre-targeting binding handle.
FIG.8 shows the targeting labeling effect of a combination probe formed by KA001 as an immune-cell-targeting probe.
FIG.9 shows the targeting labeling effect of a combination probe formed by KA004 as an immune-cell-targeting probe.
FIG. 10 shows the drug-targeting delivery effect of the targeting polypeptide with biotin as the pre-targeting binding handle after intraperitoneal injection into peritoneal free macrophages.
FIG.11 shows validation test results of drug delivery of a targeting polypeptide with biotin as the pre-targeting binding handle via mesenteric macrophages after intraperitoneal injection.
FIG.12 shows the drug-targeting delivery effect of the combination probe via pulmonary inhalation.

### DETAILED DESCRIPTION

In order to make the objectives, technical solutions and technical effects of the present disclosure clearer, the present disclosure is further described in detail below in conjunction with specific embodiments. It should be understood that the specific embodiments described in this specification are only for explaining the present disclosure and are not intended to limit the present disclosure.

Unless otherwise specified, the experimental materials and reagents used are conventional consumables and reagents that can be obtained commercially.

### Example 1 Polypeptides that target and recognize immune cells (targeting polypeptides) and antibodies therefor

The polypeptides used in the examples of the present disclosure were synthesized by conventional solid phase polypeptide chemical synthesis using a CEM fully automatic microwave polypeptide synthesizer according to the instructions provided by the instrument supplier.

The polypeptides targeting and recognizing immune cells in the examples of the present disclosure can refer to the prior patents CN 110885805 A, CN 111051504 A and CN 110760491 A, including but not limited to the sequences shown in Table 1 below.

**Table 1 Polypeptides targeting immune cells from different animal sources**

| Sequence number | Sequence | Species |
|---|---|---|
| SEQ ID NO:1 | LQPDPGAVAAAAVLRAVLEGLQG-**C**-X | *Gallus gallus* |
| SEQ ID NO:2 | DQPDPGAVAAAAIFRAILEVLQTKAA-**C**-X | *Rattus norvegicus* |
| SEQ ID NO:3 | DQPDPGAVAAAAILRTILEVLQSQGV-**C**-X | *Canis lupus* |
| SEQ ID NO:4 | DQPDPGAVAAAAILRAILEVLQSQGA-**C**-X | *Sus scrofa* |
| SEQ ID NO:5 | DQPDPGAVAAAAILRAILEVLQSQGA-**C**-X | *Bos taurus* |
| SEQ ID NO:6 | EQPDPGAVAAAAILRAILEVLQS-**C**-X | *Macaca mulatta* |
| SEQ ID NO:7 | LDQPDPGAVAAAAIFRAILEVLQTQGA-**C**-X | *Mus musculus* |
| SEQ ID NO:8 | AILEVLQS-**C**-X | *Homo sapiens* |
| SEQ ID NO:9 | LRAILEVLQS-**C**-X | |
| SEQ ID NO:10 | ILRAILEVLQS-**C**-X | |
| SEQ ID NO:11 | AAILRAILEVLQS-**C-**X | |
| SEQ ID NO:12 | EQPDPGAVAAAAILRAILEVLQS-**C**-X | |
| SEQ ID NO: 13 | PGAVAAAAILRAILEVLQS-**C**-X | |
| SEQ ID NO:14 | KNMEAGRASYISSARLEQPDPGAVAAAAILRAILEVLQS-**C**-X | |
| SEQ ID NO:15 | AVLEVLQG-**C**-X | Modified sequence |
| SEQ ID NO:16 | VLRAVLEVLQG-**C**-X | |
| SEQ ID NO:17 | EQPDPSAVAAAAILRAILEVLQG-**C**-X | |
| SEQ ID NO:18 | LQPDPSAVAAAAVLRAVLEVLQG-**C**-X | |

The underlined and bolded part (i.e., **C**) represents an adapter sequence, and X represents a marker, a tag, or a carrier part, where the carrier includes OVA, BSA, and KLH.

The modified polypeptide targeting and recognizing immune cells (targeting polypeptide) is used as an immune antigen to prepare an antibody. The antibody includes monoclonal antibodies and polyclonal antibodies. In addition, the method for preparing antibodies can be carried out with reference to the conventional technical manual in the art (Howard, Gary C., and Matthew R. Kaser, eds. Making and using antibodies: a practical handbook. CRC press, 2013.).

In this example, the polypeptide used to prepare the antibody is specifically LDQPDPGAVAAAAIFRAILEVLQTQGA (SEQ ID NO: 7) connected to BSA (i.e., X is BSA) via a linker sequence Cys (C), referred to as LC28-BSA. The antibody is a monoclonal antibody. Of course, those skilled in the art need to understand that this example is only illustrative, and the antibodies claimed in the present disclosure are not limited to those shown in this example. Those skilled in the art can prepare antibodies for the above-mentioned other polypeptides based on common sense and technical means.

In this example, the method for preparing antibodies based on LC28-BSA specifically includes the following steps:

### (1) Animal immunization:

For the first immunization, LC28-BSA emulsified with Freund's complete adjuvant was injected into the subcutaneous abdomen and footpads at different sites of Balb/C mice, with each mouse receiving 50-100 µg and a total dose of 0.2 mL per mouse. The second immunization was carried out 21 days later, and Freund's incomplete adjuvant was used for the second immunization, with a dose of 25-50 µg/0.5 mL/mouse. After the second immunization, subsequent immunizations were given every 2 weeks. After the fourth immunization, serum was collected for ELISA testing, and fusion could be performed when the titer reached 1: 20,000.

Three days prior to fusion, 50 µg of the corresponding targeting recognition peptide (LC28) was injected intraperitoneally for sprint immunization.

### (2) Cell fusion:

The immunized mice were killed, and the spleens were taken out, ground and crushed, and macromolecular impurities were removed with cell sieves. The spleen cells were then washed several times with 1640 culture medium, for cell counting. SP2/0 cells (mouse myeloma cells) in good condition in logarithmic growth phase were taken and mixed with the spleen cells from immunized mice at a ratio of 1: 5-10. After thorough mixing, the mixture was centrifuged, with the supernatant discarded. 1 mL of 45% PEG solution was added to the precipitated cells, with gently rotating the centrifuge tube to allow the cells to fully and evenly exposed to the PEG, and then left for 1 min. 1, 2, 3, 4, 5, and 10 mL of 1640 culture medium were added at intervals of 2 min to terminate the PEG reaction, and the mixture was allowed to stand for 10 min. The cell pellets were collected by centrifugation at 800 rpm/5 min. The cells were resuspended in HAT medium and inoculated into 96-well plates for continued culture. When the hybridoma cells grew to about 1/10 of the well area, ELISA experiments were performed to screen positive hybridoma cells. After four subclonings, a cell line stably secreting monoclonal antibodies was obtained.

The cell line was acclimated with serum-free medium, the culture supernatant was collected, and the antibody in the supernatant was enriched and purified using protein A/G magnetic beads to obtain a monoclonal antibody (KA-001).

The prepared monoclonal antibody (KA-001) was sequenced, and its heavy chain amino acid sequence (Mouse IgG1 subtype) was:

DVKIVESGGGLVKLGGSLKLSCAASGFTFSRNYMSWVRQTPEKRLELVAGINNNGGS
TYYPDTVKGRFTISRDNAKNTLYLQMSSLRSEDTAIYYCAKRGFITTVGAMDYWGQGISV
TVSSAKTTPPSVYPLAPGSAAQTNSMVTLGCLVKGYFPEPVTVTWNSGSLSSGVHTFPAVL
QSDLYTLSSSVTVPSSTWPSETVTCNVAHPASSTKVDKKIVPRDCGCKPCICTVPEVSSVFIFP
PKPKDVLTITLTPKVTCVVVDISKDDPEVQFSWFVDDVEVHTAQTQPREEQFNSTFRSVSEL
PIMHQDWLNGKEFKCRVNSAAFPAPIEKTISKTKGRPKAPQVYTIPPPKEQMAKDKVSLTC
MITDFFPEDITVEWQWNGQPAENYKNTQPIMDTDGSYFVYSKLNVQKSNWEAGNTFTCSV
LHEGLHNHHTEKSLSHSPGK (SEQ ID NO: 19).

The bolded and underlined parts are the three complementary regions (CDRs) in the heavy chain variable region of KA-001.

The nucleotide sequence corresponding to the heavy chain of KA-001is:

5’-GATGTGAAGATAGTGGAAAGTGGTGGTGGATTGGTCAAACTTGGGGGGTCCCTGAAA
CTCAGCTGCGCTGCCAGCGGCTTCACATTTAGTAGAAACTACATGTCCTGGGTGAGGCA
AACGCCAGAGAAGCGGCTGGAGCTTGTAGCTGGCATCAACAATGGAGGCAGCACCTAC
TATCCTGACACAGTCAAAGGACGCTTCACCATATCCAGAGACAATGCAAAGAATACCCT
GTATCTGCAGATGTCATCTTTACGATCAGAAGATACTGCAATCTATTACTGTGCCAAGAG
GGGTTTTATTACAACTGTGGGGGCCATGGACTACTGGGGCCAGGGCATTTCTGTTACTGT
GTCCTCCGCCAAGACCACACCACCTTCCGTCTATCCCTTGGCACCAGGATCAGCCGCCC
AAACTAACAGTATGGTGACACTGGGATGCCTGGTTAAGGGCTATTTCCCTGAGCCAGTG

ACAGTGACTTGGAACAGTGGTAGCCTGAGTTCAGGGGTCCACACTTTCCCCGCTGTCTT
GCAGAGTGATCTGTATACCCTCAGCTCCTCAGTGACCGTTCCTAGCAGTACGTGGCCTTC
CGAGACTGTGACATGTAATGTAGCCCATCCCGCTTCATCTACCAAGGTGGATAAGAAGAT
TGTTCCACGGGACTGCGGGTGTAAACCGTGTATTTGTACTGTACCCGAGGTGAGTTCCG
TTTTCATTTTCCCTCCCAAGCCCAAAGATGTTCTCACAATTACTCTGACACCAAAAGTTA
CATGCGTGGTGGTGGACATCAGCAAGGACGATCCTGAGGTGCAGTTTTCCTGGTTTGTT
GATGACGTCGAAGTCCACACTGCACAGACCCAGCCAAGGGAAGAACAGTTCAATAGTA
CATTCAGATCCGTGTCCGAATTGCCGATTATGCATCAGGACTGGCTCAATGGCAAAGAAT
TTAAGTGTAGAGTGAACTCTGCCGCCTTTCCTGCTCCTATCGAAAAGACAATCAGCAAG
ACTAAGGGCAGACCTAAAGCCCCCCAAGTGTATACTATTCCACCCCCCAAAGAACAGATG
GCCAAGGACAAAGTAAGTTTGACCTGTATGATCACTGACTTCTTCCCAGAAGATATCACT
GTGGAATGGCAGTGGAACGGACAGCCTGCGGAGAACTATAAAAATACTCAGCCCATTAT
GGACACAGACGGGTCTTACTTCGTCTACAGTAAACTGAATGTTCAGAAAAGTAATTGGG
AGGCCGGCAATACATTCACCTGTTCAGTGCTGCATGAGGGGCTTCACAACCACCACACT
GAGAAATCCCTTAGCCACAGCCCTGGCAAGTAA-3' (SEQ ID NO: 20).

The light chain amino acid sequence (Mouse Kappa subtype) of KA-001is:

DIQMTQTTFSLSASLGDRVTISCRASQDISNYLNWYQQIPDGTVKLLIYYTSRLHSGVP
SRFSGSGSGTDYSLTISNLEQEDIATYFCQQVHTLPYTFGGGTKLEMKRADAAPTVSIFPPSS
EQLTSGGASVVCFLNNFYPKDINVKWKIDGSERQNGVLNSWTDQDSKDSTYSMSSTLTLT
KDEYERHNSYTCEATHKTSTSPIVKSFNRNEC (SEQ ID NO: 21).

The bolded and underlined parts are the three complementary regions (CDRs) in the light chain variable region of KA-001.

The nucleotide sequence corresponding to the light chain of KA-001is:

5’-GACATTCAGATGACCCAGACGACCTTCAGCCTCTCTGCATCCCTTGGAGACAGAGTC
ACCATCAGCTGTCGGGCCAGCCAAGATATATCAAATTATCTGAACTGGTACCAGCAGATC
CCAGATGGCACTGTGAAGCTGCTTATCTACTACACATCACGCCTACATTCTGGTGTTCCT
TCCAGGTTTTCTGGGAGTGGCAGTGGGACAGACTATTCCCTCACCATTAGCAACCTGGA
GCAGGAAGATATTGCTACTTACTTCTGCCAGCAAGTGCACACACTGCCCTATACTTTTGG
AGGTGGCACCAAATTGGAGATGAAACGGGCCGATGCTGCTCCTACCGTCAGTATCTTCC
CACCTTCAAGCGAACAGCTTACCAGTGGCGGGGCATCTGTGGTGTGCTTCCTGAATAAC
TTTTACCCAAAGGATATAAACGTGAAGTGGAAGATCGACGGCAGCGAGCGGCAAAATG
GAGTGTTGAATAGTTGGACTGACCAGGACTCTAAGGACTCCACCTACTCTATGTCATCTA
CACTTACACTGACAAAGGATGAGTACGAACGGCACAACTCCTATACATGCGAAGCTACC
CATAAGACCAGTACCAGCCCCATTGTGAAGTCATTCAATAGAAATGAGTGCTAA-3’ (SEQ

ID NO: 22).

### Example 2 Preparation and purification of antibodies based on genetic engineering

In the present disclosure, the acquisition of antibodies is not limited to a specific method. In this example, the preparation method of the above-mentioned KA-001 antibody based on genetic engineering is exemplified.

The specific steps are as follows:

### (1) Primer design:

In this example, the following primers were used for preparing the KA-001 antibody by genetic engineering.

KA-001 Heavy Chain Primer Pair:
Upstream primer KA-001H-F: 5'-GGCCTCCGGACTCTAGAGGATCCGCCACCATGGAGACAGACACACTCCTGCT-3' (SEQ ID NO: 23);
Downstream primer KA-001H-R: 5'-CTCGAGCTAAGCTTCGAATTCTTACTTGCCAGGGCTGTG-3' (SEQ ID NO: 24).

KA-001 Light Chain Primer Pair:
Upstream primer KA-001L-F: 
5'-CAGAATTCGAAGCTTAGCTCGAGAGCCACCATGGAGACAGACACACTCCTGCTATGG
-3' (SEQ ID NO: 25);
Downstream primer KA-001L-R: 5'-CAGAGGTTGATTGTCGAGATATCAAACTCATTACTAACCGGT-3' (SEQ ID NO: 26).

### (2) Obtaining the target sequence:

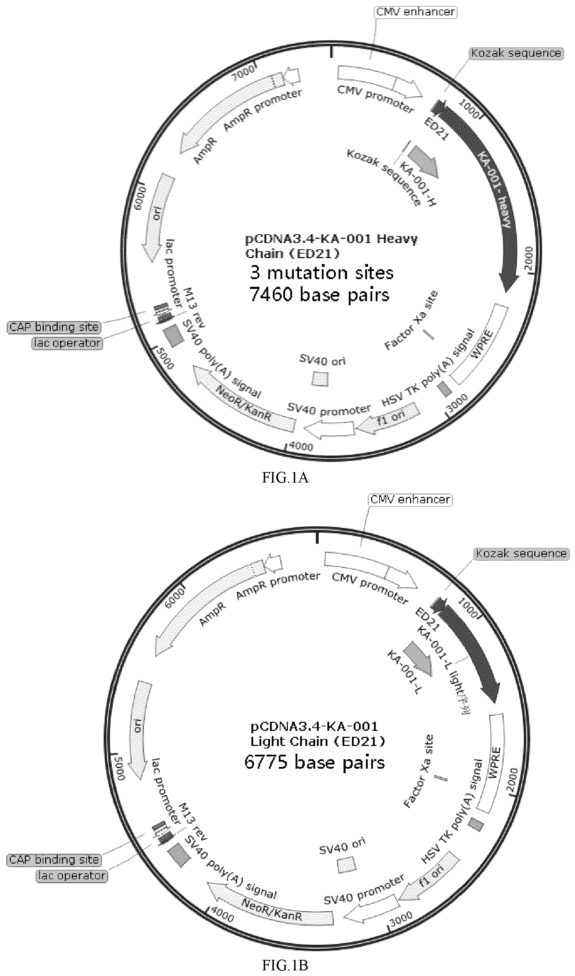

Taking a plasmid containing nucleic acid molecules encoding the sequences shown in SEQ ID NO: 19 and SEQ ID NO: 21 (the plasmid map was shown in FIG. 1, and the plasmid was named pCDNA3.4) as a template, the target sequence was amplified based on the primers shown in SEQ ID NO: 23 to SEQ ID NO: 26 and PrimeSTAR high fidelity enzyme. The amplification system and conditions can be adjusted according to the actual situation and the instructions for use of the relevant kit.

The amplified products were verified by 1% agarose gel electrophoresis. After confirmation, the target sequence was recovered.

### (3) Enzyme digestion and ligation of target sequence and vector plasmid:

The recovered target sequence and the pcDNA3.4 vector were subjected to double digestion using restriction endonucleases NotI and XhoI. The restriction endonuclease system was constructed by using a conventional restriction endonuclease system in the art or referring to instructions for use of the restriction endonucleases. After double enzyme digestion, the product was verified by 1% agarose gel electrophoresis. After confirmation, the target sequence and linearized vector after restriction digestion were recovered.

The target sequence and the linearized vector were connected to obtain a recombinant vector.
(4) The recombinant vector obtained in (3) was transformed into DH5α competent cells, and positive cellswere screened (verified by PCR and sequencing) to extract the plasmid.
(5) The plasmid extracted in (4) was transfected into expression vector cells (HEK-293) using KPM cell transfection buffer and TA-293 transfection reagent according to transient transfection technology. After being transfected for 24 hours, a cell protein expression enhancer (KE-293) and a transient transfection nutrient additive (KT-Feed 50×) were added to promote expression. The cell supernatant was collected after being transfected for 6 days, and the expression level of the target protein (KA-001) was determined using ELISA technology.
(6) The target protein in the cell supernatant was purified using liquid chromatography: the cell supernatant was centrifuged at 8,000 rpm for 30 min and filtered through a 0.45 µm filter membrane. The filtrate was passed through 1 mL chromatography column at a flow rate of 1 mL/min. The column was washed with 10-15 column volumes of PBS to remove impurities and then eluted with a 0.1 M glycine solution at pH 3.0 to recover the target antibody based on the absorption peak. The recovered antibodies were neutralized with 1/10 volume of 1M Tris-HCl, pH 8.5. After ultrafiltration and concentration, the antibody concentration was determined by protein electrophoresis.

In addition, according to the method described in the above example, the KA-004 antibody was prepared by the same genetic engineering method, where the polypeptide corresponding to the KA-004 antibody is specifically EQPDPGAVAAAAILRAILEVLQS (SEQ ID NO: 12) connected to BSA (i.e., X is BSA) by a linker sequence Cys (C), referred to as EC24-BSA.

### Example 3 Specific binding effect of KA-001 and KA-004 on VLQ

The specific binding effect of KA-001 and KA-004 on VLQS was determined by ELISA method. The specific steps were as follows:
Peptide segments KL19 (KEQPDPGAVAAAAILRAIL (SEQ ID NO: 27)), KL22 (KEQPDPGAVAAAAILRAILEVL (SEQ ID NO: 28)), and KS24 (KEQPDPGAVAAAAILRAILEVLQS (SEQ ID NO: 29)) were taken, where KL19 does not contain VLQS, KL22 only contains VL, and KS24 contains VLQS. The three peptide segments were used as coating antigens, and the binding of KA-001 and KA-004 to the three peptides was detected by indirect ELISA.

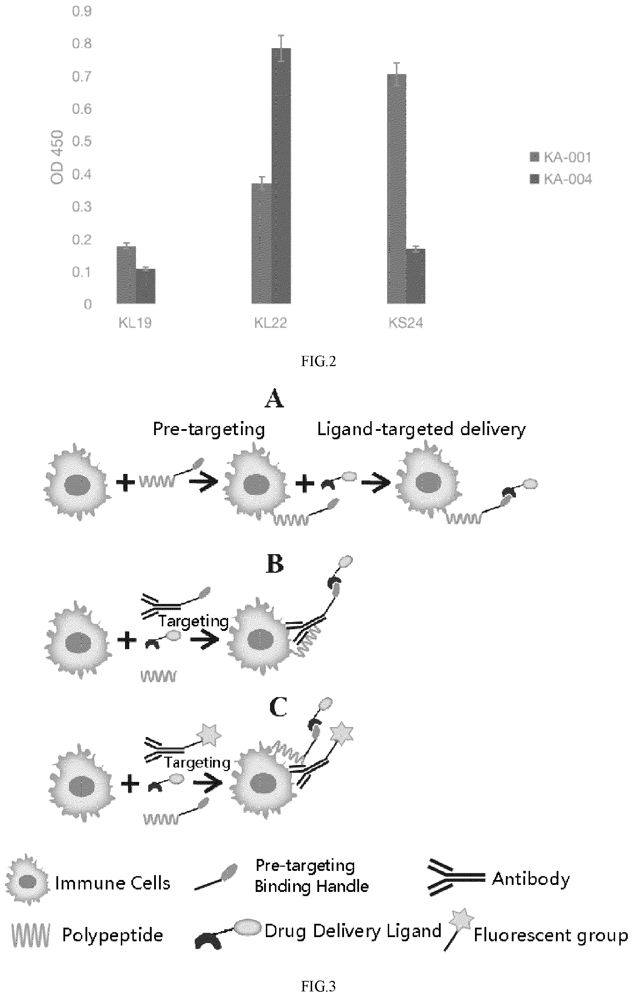

As shown in FIG. 2, the results showed that KA-001 and KA-004 specifically bound to KS24 (the last two amino acids in VLQS) and its adjacent epitope KL22 (the first two amino acids in VLQ), respectively, but neither bound to KL19, indicating that the recognition epitope of the antibody in the above embodiment is an amino acid sequence with more than 50% identity in VLQ. The inventors have found that after the antibody recognizing the epitope forms a self-assembled probe with the targeting polypeptide, it has a more sensitive recognition ability for immune cells.

### Example 4 Application of immune-cell-targeting probes formed based on different combinations of targeting polypeptides or their antibodies in animals

In example of the present disclosure, the immune-cell-targeting probe includes at least one of the following (1) to (2), or a combination thereof with a drug delivery ligand:
(1) a targeting polypeptide of Example 1 connected with a pre-targeting binding handle; and
(2) an antibody or a functional fragment thereof prepared from the targeting polypeptide as an immune antigen and connected with a pre-targeting binding handle.

The above-mentioned drug delivery ligand is connected with a linker, which can specifically bind to the pre-targeting binding handle, and after the connection, it does not affect the binding stability of (1) or (2) with immune cells.

In example of the present disclosure, the targeting polypeptide in (1) specifically includes at least one amino acid sequence having at least 95% identity with the amino acid sequence shown in SEQ ID NOs: 1 to 18. The antibodies or functional fragments thereof mentioned in (2) include any antibodies or functional fragments thereof that use the targeting polypeptide as an immune antigen and are capable of recognizing or binding to the targeting polypeptide. The antibodies or functional fragments thereof may be connected with a reporter group or other modifiers that do not affect their recognition or binding ability, including but not limited to colorimetric enzymes, fluorescent labeling groups, chemiluminescent labeling groups, isotopes or magnetic functional groups.

Both (1) and (2) can be used alone, and (2) can also be used in combination with (1) as a reinforcing component. The combination of (1) and (2) is based on the self-assembly produced by the specific recognition of antigen-antibody, thereby forming a combination probe. In the combination probe, the pre-targeting binding handle can be connected to any one of the targeting polypeptides or antibodies alone, or the pre-targeting binding handle can be connected to both the targeting polypeptides or antibodies. The number of the pre-targeting binding handle can be adjusted according to actual usage requirements. In the example of the present disclosure, in the combination probe, the pre-targeting binding handle is only connected to the antibody, with one handle connected. Of course, it should be understood that this example should not be construed as limiting the combination probe in the present disclosure. In a specific product, (1), (2) and the drug delivery ligand can be stored separately and used in sequence according to the situation so that they can self-assemble to form a combination probe.

In addition, the above-mentioned pre-targeting binding handle and the linker have a specific binding relationship, specifically including mutually corresponding substances with specific binding ability such as biotin-avidin system, specific complementary sequences, antigen-antibody, etc.

Based on the types of immune-cell-targeting probes in this example, the recognition modes of the immune-cell-targeting probes in the present disclosure can be divided into three types. As shown in FIG. 3 , the first mode is to use the targeting polypeptide connected with the pre-targeting binding handle in (1) to perform pre-targeting recognition on the immune cells, and then add the drug delivery ligand to recognize and bind to the pre-targeting binding handle in (1) that has been fixed on the immune cells based on the linker on the drug delivery ligand, thereby performing pre-targeting directional drug delivery to the immune cells. The second mode is to pre-mix and self-assemble the targeting polypeptide in (1), the antibody or its functional fragment connected with the pre-targeting binding handle in (2), and the drug delivery ligand *in vitro,* and then inject them into the body to form a combination probe for targeted recognition of immune cells, thereby delivering drugs to immune cells in a directional manner. The third mode is to pre-mix and self-assemble the targeting polypeptide connected with the pre-targeting binding handle in (1), the antibody or its functional fragment connected with a fluorescent group in (2), and the drug delivery ligand *in vitro,* and then inject them into the body to form a combination probe for targeted recognition of immune cells, thereby delivering drugs to immune cells in a directional manner.

### Example 5 Targeting labeling effect of polypeptide-targeting antibodies as immune-cell-targeting probes

In the examples of the present disclosure, the antibodies prepared based on the polypeptides targeting and recognizing immune cells (targeting polypeptides) in the above examples as immune antigens all have the function of targeting and marking immune cells. In this example, the KA-001 monoclonal antibody prepared in the above example was used as an example, and the B cells and granulocytes obtained from the peripheral blood of Kunming mice were used as target objects to verify the effect.

Of course, those skilled in the art need to understand that this example is only used as an example, and the generation of this effect is not limited to the KA-001 monoclonal antibody.

The specific test steps are:
A healthy Kunming mouse was taken to collect peripheral blood. 50 µL of mouse peripheral blood was taken into EP tubes, 50 µL of 1×PBS was added for dilution, and then 2 µL of KA-001 antibody prepared in the above example was added, and incubated at 4°C for 30 min. After the incubation, the red blood cells in the whole blood were lysed using a conventional red blood cell lysis reagent in the art according to the instructions, the lysed whole blood was resuspended in 100 µL of 1× PBS, and 0.5 µL/tube of CD19 antibody (anti-mouse CD19 Antibody), Gr-1 antibody (anti-mouse Gr-1 Antibody) (both antibodies were purchased from Biolegend) and 1 µL/tube of PE-labeled (streptavidin) (Strep-PE) were added and incubated at room temperature for 1 hour. After incubation, the cells were washed twice with 1×PBS and analyzed by flow cytometry. The peripheral blood samples of mice without any treatment were used as blank control (NC).

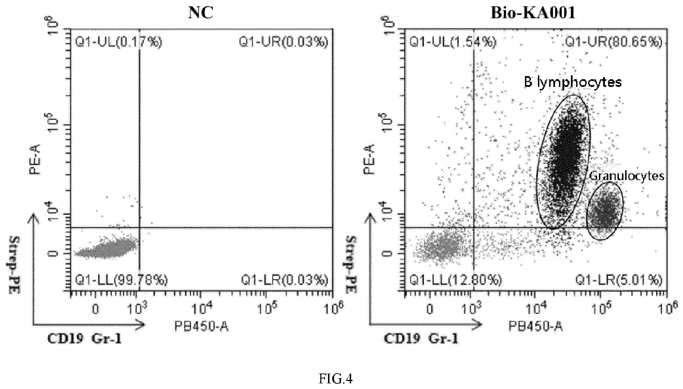

The results were shown in FIG. 4.

It can be found that after treatment with KA-001 monoclonal antibody, B cells and granulocytes in the peripheral blood of Kunming mice can be accurately identified based on flow cytometric analysis.

### Example 6 Targeting labeling effect of the targeting polypeptide connected with pre-targeting binding handle as immune-cell-targeting probe

*(1) In vivo* labeling of peritoneal macrophages by targeting polypeptides connected with pre-targeting binding handles:
   In this example, the targeting polypeptides KS24 (amino acid sequence: KEQPDPGAVAAAAILRAILEVLQS (SEQ ID NO: 29)) and KS29 (amino acid sequence: KSSARLEQPDPGAVAAAAILRAILEVLQS (SEQ ID NO: 30)) were used. The pre-targeting binding handle was biotin. The biotinylation steps of the targeting polypeptides were as follows: the biotinylation of targeting polypeptides was carried out by adding biotinylated amino acids during peptide synthesis, so that the biotinylation sites can be controlled. Two biotinylated targeting polypeptides (bio-KS24 and bio-KS29) were respectively connected to phycoerythrin-labeled streptavidin (Strep-PE), where streptavidin served as a linker, and the phycoerythrin was connected through the specific binding of the linker to the pre-targeting binding handle (biotin-avidin system) to the targeting polypeptide for immune cell labeling and tracing. The mixtures were incubated together at room temperature for 1 h. In a 100 µL reaction system, the final concentration of the biotinylated targeting polypeptide was 10 µM, and 1 µL of Strep-PE was added. After the incubation, two groups of healthy female Kunming mice (3 mice in each group) were selected. After the two mixed systems were diluted to 4 times of the original volume with 1×PBS (i.e., 100 µL of the mixed system was diluted to 400 µL), different groups of experimental mice were injected intraperitoneally. The injection volume for each mouse was 400 µL. The mice were killed after injection of 4 h, and peritoneal cells were extracted and counterstained with F4/80-APC antibody. After incubation, the cells were washed twice with 1× PBS and analyzed using flow cytometry.

In addition, a group of healthy female Kunming mice without treatment was set up as blank control (NC).

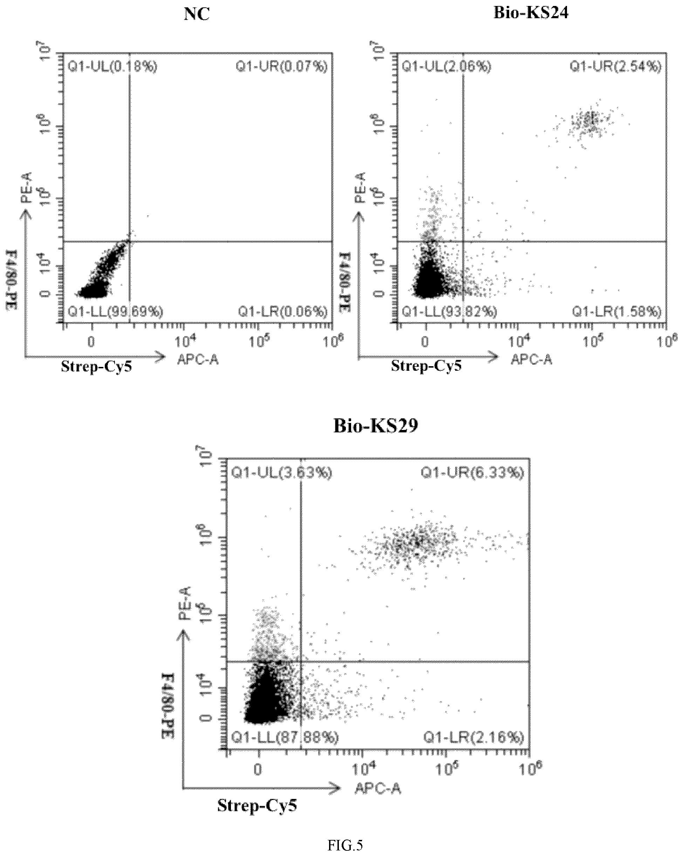

The results were shown in FIG. 5.

It can be found that the targeting polypeptide connected with the pre-targeting binding handle constructed based on biotinylated KS24 and KS29 can selectively recognize macrophages in peritoneal cells of mice, and the recognition pattern was consistent with that of the F4/80 antibody, indicating that the targeting polypeptide connected with the pre-targeting binding handle can be used as an immune-cell-targeting probe to accurately and stably identify immune cells. Moreover, the targeting effect of the targeting polypeptide connected with the pre-targeting binding handle was not affected by the administration method. Even when administered via intraperitoneal injection, the targeting polypeptide remained stable *in vivo* and demonstrated excellent recognition efficiency over time.

(2) Labeling of peripheral blood with the targeting polypeptide connected with pre-targeting binding handle:
Healthy Kunming mice were taken and killed by cervical dislocation, and their peripheral blood was collected using anticoagulant blood vessels.

KS24 and KS29 were biotinylated according to the method described in (1) to obtain targeting polypeptides bio-KS24 and bio-KS29 connected with pre-targeting binding handle. The targeting polypeptides bio-KS24 and bio-KS29 each connected with the pre-targeting binding handle were added to 50 µL of peripheral blood samples, respectively, to be diluted to 100 µL with 1×PBS so that the concentration of the targeting polypeptides bio-KS24 and bio-KS29 connected with the pre-targeting binding handle in the solution was 10 µM, and the mixture was incubated at 4°C for 30 min. After the incubation, red blood cells in the peripheral blood were lysed using a conventional red blood cell lysis reagent in the art according to the instructions, washed twice with 1×PBS, resuspended with 100 µL of 1×PBS, and 1 µL/tube of phycoerythrin-labeled Strep-PE, 0.5 µL/tube of anti-mouse CD19 Antibody, and 0.5 µL/tube of FITC anti-mouse CD5 Antibody were added, and incubated at room temperature for 1 h. After the incubation, the cells were washed twice with 1× PBS and analyzed by flow cytometry.

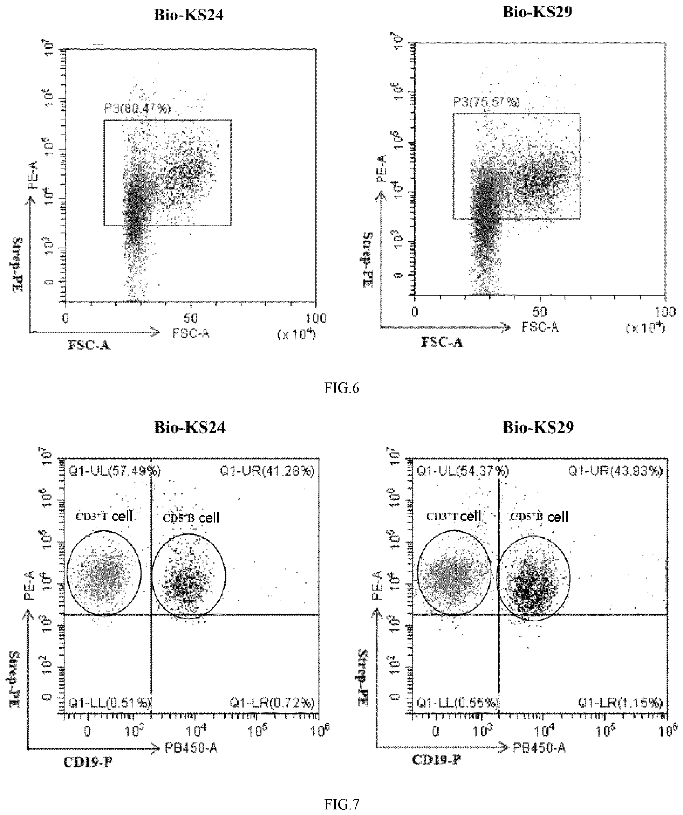

The results were shown in FIGs. 6 and 7.

It can be found that the targeting polypeptides bio-KS24 and bio-KS29 connected with the pre-targeting binding handle can stain a subset of B lymphocyte population and granulocyte population in the peripheral blood of Kunming mice, and it can be seen from the results in FIG. 6 that the labeled B lymphocyte population was identified as CD5⁺ B lymphocytes.

### Example 7 Targeting labeling effect of combination probe as immune-cell-targeting probe

In this example of the present disclosure, the inventors used the combination probe as the immune-cell-targeting probe to target, identify or label immune cells. In this combination probe, the antibody connected to the pre-targeting binding handle or its functional fragment is biotin-modified KA-001 (the pre-targeting binding handle is biotin), and the targeting peptide is ES23 (with an amino acid sequence: EQPDPGAVAAAAILRAILEVLQS (SEQ ID NO: 31)) or SS28 (with an amino acid sequence: SSARLEQPDPGAVAAAAILRAILEVLQS (SEQ ID NO: 32)).

Of course, those skilled in the art need to understand that this example is merely illustrative, and the generation of the effect is not limited to KA-001 or KA-004 monoclonal antibodies.

The specific experimental steps are as follows:
KA-001 and KA-004 were modified with biotin to obtain KA-001 and KA-004 antibodies connected with pre-targeting binding handles. The specific steps are as follows: KA-001/KA-004 antibodies were first purified with Protein G and then dialyzed overnight at 4°C in a 0.1 M NaHCO₃ solution, pH 8.0. Biotin-N-hydroxysuccinimide ester was dissolved in DMSO to form a solution with a concentration of 1 mg/mL, and then 200 µL of Biotin-N-hydroxysuccinimide ester was added per 1 mg of antibody to a final volume of 1 mL. After incubation at room temperature for 4 h, the mixture was dialyzed in PBS with pH 7.4 for 24 h at 4°C. The dialyzed antibody was centrifuged at 3,000 rpm for 30 min, the precipitate was discarded, and the supernatant was the biotin-labeled KA001 antibody. Two groups of healthy Kunming mice were selected and their peripheral blood was collected. For each sample, 50 µL of mouse peripheral blood was collected into an EP tube, and a targeting polypeptide (ES23, SEQ ID NO: 31 or SS28, SEQ ID NO: 32) with a final concentration of 10 µM and a KA-001/KA-004 antibody connected with a pre-targeting binding handle with a final concentration of 2 µL/100 µL total system were added. After thorough mixing, the mixture was incubated at 4 °C for 30 min. A 0.4% (m/m) ammonium chloride solution (prepared with pure water and filtered through a 0.1 µm filter before use) was added to the peripheral blood mixture (containing the targeting polypeptide and the antibody connected with the pre-targeting binding handle) in a volume ratio of 1: 25 (ammonium chloride: peripheral blood) to lyse the peripheral blood at room temperature for 10 min, centrifuged at 400 g for 5 min to collect the precipitated cells, The cells were resuspended in PBS, and centrifuged at 400 g for 5 min, and the cells were washed twice. After resuspending the cells in 100 µL of PBS, the cells were analyzed using a flow cytometer.

The group without adding the targeting peptide was used as the control, and a blank group was also set up. The experiment was repeated twice.

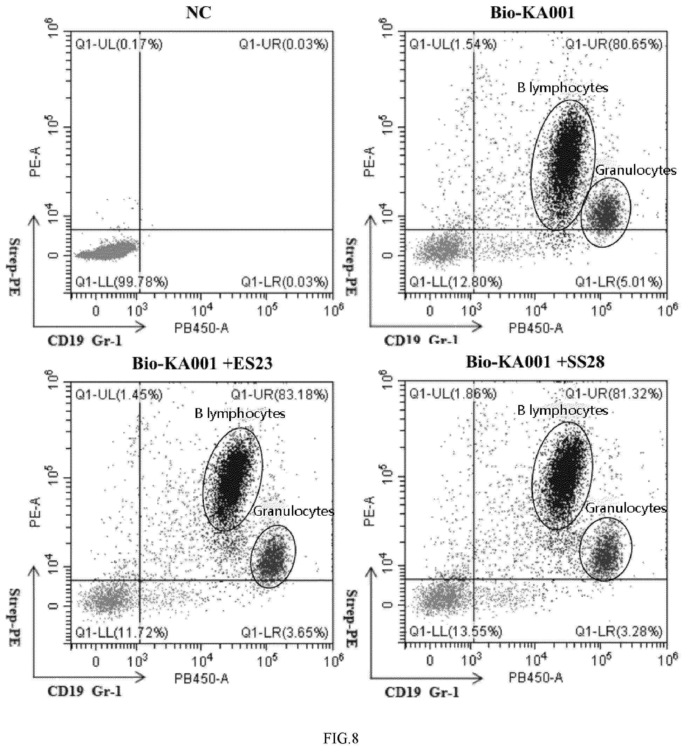

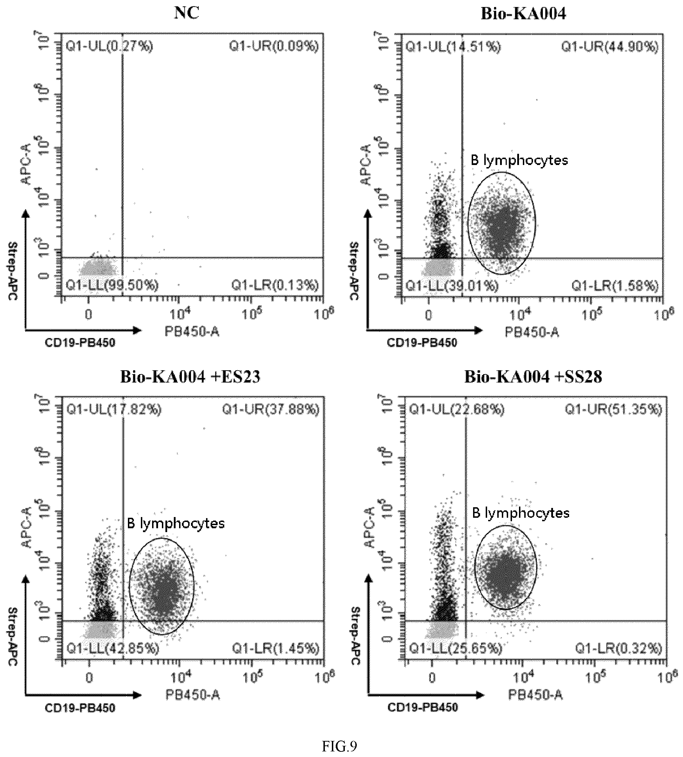

The results were shown in FIGs. 8 and 9.

It was found that through two repeated experiments, the group using only the antibody connected with the pre-targeting binding handle was able to effectively identify monocytes, neutrophils and B lymphocyte populations in the peripheral blood cells of healthy mice. Upon further addition of the targeting polypeptide, the combination probe formed by self-assembly of the targeting polypeptide and the antibody connected with the pre-targeting binding handle had a significantly enhanced recognition effect on monocytes, neutrophils and B lymphocytes in the peripheral blood cells of healthy mice, and the percentage of positive cells increased, indicating that the binding efficacy of the combination probe formed by their self-assembly was significantly improved compared with using only the antibody connected with the pre-targeting binding handle.

### Example 8 Application of combination probes in pre-targeting drug delivery systems

In this example, the inventors tested the use of the combination probe as a targeted drug delivery carrier through various administration methods.
(1) Targeted drug delivery effect of targeted polypeptides with biotin as pre-targeting binding handle after intraperitoneal injection:
   In this example, the inventors used two targeting polypeptides (bio-KS24 and bio-KS29) connected with pre-targeting binding handles as examples to demonstrate the effect of combining the targeting polypeptides connected with the pre-targeting binding handles with drug delivery ligands in targeted drug delivery. Of course, those skilled in the art need to understand that this example is merely illustrative, and the generation of this effect is not limited to the above two targeting polypeptides.

The specific experimental steps are as follows:
Biotinylated targeting polypeptides bio-KS24 and bio-KS29 (i.e., targeting polypeptides connected with the pre-targeting binding handles) were prepared according to the steps described in the above examples.

The targeting polypeptides (bio-KS24 and bio-KS29) connected with the pre-targeting binding handles were prepared, respectively, with a final concentration of 10 µM and a final volume of 100 µL. Then, the mixture was diluted with PBS to 4 times of the original volume (i.e., 100 µL of the mixed system was diluted to 400 µL), and then injected into Kunming mice intraperitoneally, with an injection volume of 400 µL per mouse. After the injection of the targeted polypeptide for 3 h, 5 µL Trep-PE was diluted into 100 µL of 1× PBS and intraperitoneally injected into mice. The mice were killed 1 h after the injection, and free peritoneal cells and mesentery were extracted and counterstained with F4/80-APC antibody. After incubation, the cells were washed twice with 1× PBS and observed under a fluorescence microscope.

Untreated healthy mice were used as controls.

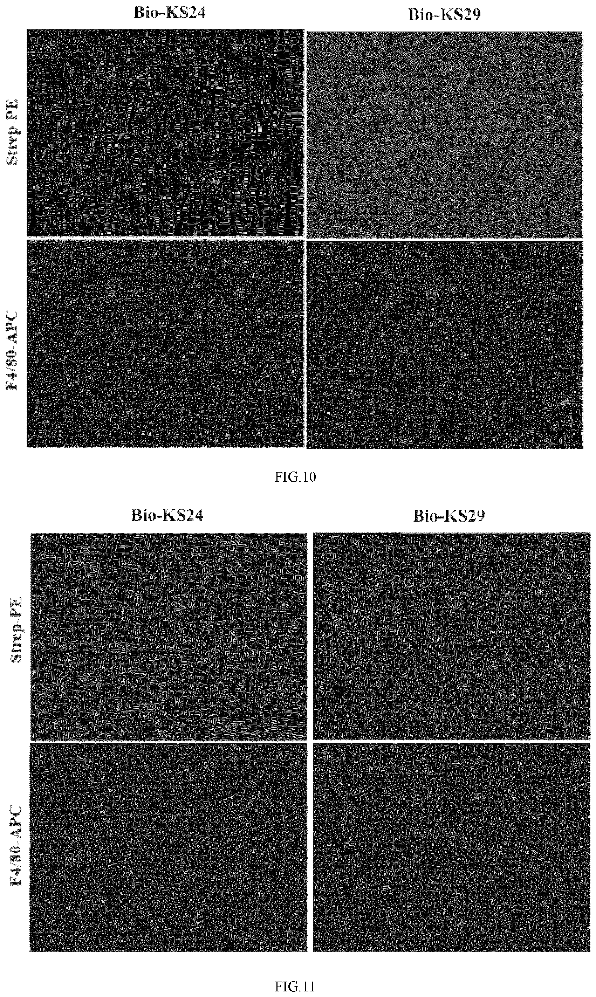

The results were shown in FIGs. 10 and 11.

The results showed that intraperitoneally injected bio-KS24 and bio-KS29 could pre-label macrophages, and their staining results were similar to those of F4/80-APC. In addition, intraperitoneally injected bio-KS24 and bio-KS29 could pre-label macrophages on the mesentery, and the staining effect was basically similar to that of F4/80-APC. The above results indicated that the combination probes in the examples of the present disclosure can accurately pre-target the macrophages in the peritoneal cavity through intraperitoneal injection, and then by modifying the delivery drug with avidin (phycoerythrin was used to simulate the mounted delivery drug in this example), and utilizing the specific recognition effect of biotin-avidin, the secondary targeted delivery of the drug to the free macrophages in the body and the macrophages on the mesentery can be achieved.

(2) Targeted drug delivery effect of combination probes through lung inhalation:
In this example, the inventors used biotinylated KS24/KS29 as the targeting polypeptide connected with pre-targeting binding handle, and AlexaFluor647-labeled KA-001 as antibody to demonstrate the effect of the combination probe formed by combing the antibody as an enhancement component with the targeting polypeptide connected with pre-targeting binding handle in combination with a drug delivery ligand in targeted drug delivery. Of course, those skilled in the art need to understand that this example is merely illustrative, and the generation of this effect is not limited to the above-mentioned targeting polypeptides and antibodies.

In addition, bio-KS24, bio-KS29 and AlexaFluor647-labeled KA-001 were obtained by referring to the methods described in the above examples and conventional methods in the art.

Bio-KS24/bio-KS29 was taken to be added to 1×PBS for dilution. Based on 200 µL of dilution system, the final concentration of bio-KS24/bio-KS29 in the dilution system was 100 µM. 2 µL of AlexaFluor647-labeled KA-001 and 1 µL of Strep-PE (simulating a drug delivery ligand without drug loading, in which Strep-PE served as linker) were added to 200 µL of bio-KS24/bio-KS29 dilution solution. After incubation at room temperature for 1 h, 200 µL of the mixture was inhaled into the lungs of the experimental mice by pulmonary inhalation. The mice were killed after 4 h of administration, and the lungs were taken to prepare lung compression films, which were observed under a fluorescence microscope. Untreated healthy mice were used as controls.

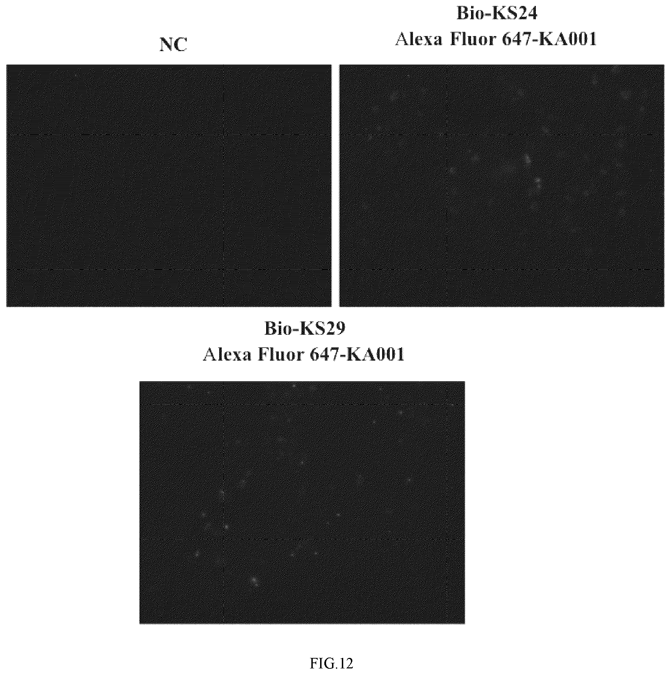

The results were shown in FIG. 12.

The results showed that after the combination probe, formed through self-assembly of bio-KS24/bio-KS29 and AlexaFluor647-KA-001, could load a drug delivery ligand using Strep-PE as a linker. Purple fluorescence could be observed in the lungs of mice after pulmonary administration, while no such fluorescence was observed in the lungs of blank control mice. This indicated that the combination probe can accurately target the immune cells in the lungs through inhalation, and by loading specific drugs onto the combination probe, targeted drug delivery can be achieved. Moreover, it can be found that due to the large molecular weight of avidin and different spatial structures of binding sites in different cells, the simultaneous injection of bio-KS24/bio-KS29 and KA-001 for self-assemble to form a combination probe and the drug delivery ligand mounted with avidin can effectively avoid the spatial structure of the polypeptide site blocking the binding of biotin and avidin after bio-KS24/bio-KS29 binds to the site. However, it is also foreseeable that when there is no risk of spatial obstruction or the drug in the drug delivery ligand has certain toxic hazards, first injecting bio-KS24/bio-KS29 and KA-001 for self-assemble to form a combination probe, and then injecting the drug delivery ligand mounted with avidin can effectively reduce the issue of poor targeting efficiency and prolonged off-target circulation of the drug in the body.

The above embodiments are preferred implementation modes of the present disclosure, but the implementation modes of the present disclosure are not limited to the above embodiments. Any other changes, modifications, substitutions, combinations, and simplifications made without departing from the spirit and principles of the present disclosure shall be equivalent replacement methods and shall be included in the protection scope of the present disclosure.

## Claims

1. An immune-cell-targeting probe, comprising the following (1), or a combination of (1) and (2):
(1) a targeting polypeptide connected with a pre-targeting binding handle; and
(2) an antibody or a functional fragment thereof produced by immunization with the targeting polypeptide in (1), wherein the antibody or the functional fragment thereof connected with the pre-targeting binding handle.

2. The immune-cell-targeting probe according to claim 1, wherein when (1) and (2) are used in combination, the immune-cell-targeting probe comprises one or more pre-targeting binding handles that are respectively or individually present on at least one of the targeting polypeptide, or the antibody or the functional fragment thereof.

3. The immune-cell-targeting probe according to claim 1, wherein the immune-cell-targeting probe is further connected with a linker and a marker.

4. The immune-cell-targeting probe according to claim 3, wherein the marker comprises a reporter group or a carrier.

5. The immune-cell-targeting probe according to claim 4, wherein the reporter group is a chromogenic enzyme, a fluorescent labeling group, a chemiluminescent labeling group, an isotope or a magnetic functional group.

6. The immune-cell-targeting probe according to claim 3, wherein the pre-targeting binding handlespecifically recognizes and binds to the linker.

7. The immune-cell-targeting probe according to claim 3, wherein a binding mode between the pre-targeting binding handleand the linker comprises biotin-avidin, complementary nucleic acid molecules and antigen-antibody.

8. The immune-cell-targeting probe according to claim 1, wherein the targeting polypeptide is a C-terminal fragment sequence of Triokinase/FMN cyclase, comprising:
(a) an amino acid sequence containing VLQmotif;
(b) an amino acid sequence having more than 80% identity with the amino acid sequence described in (a), wherein the amino acid sequence is of non-human animal origin; and
(c) a variant of the amino acid sequence described in (a) or (b).

9. The immune-cell-targeting probe according to claim 8, wherein when (1) and (2) are used in combination, the targeting polypeptide is a C-terminal fragment sequence of Triokinase/FMN cyclase containing VLQ motif.

10. The immune-cell-targeting probe according to claim 8, wherein the amino acid sequences described in (a), (b) and (c) comprise 45 or less amino acid residues, preferably 41 or less amino acid residues, and more preferably 9 to 41 amino acid residues.

11. The immune-cell-targeting probe according to claim 8, wherein the C-terminal fragment sequence of the Triokinase/FMN cyclase described in (a) comprises an amino acid sequence having more than 80% identity with amino acid sequences shown in SEQ ID NOs: 2 to18 and 29.

12. The immune-cell-targeting probe according to claim 1, wherein amino acid sequences of complementarity-determining regions (CDRs) of a heavy chain variable region of the antibody or the functional fragment thereof are as shown in amino acid sequences at positions 26 to 35, 50 to 66, and 99 to 110 of SEQ ID NO: 19, respectively; and amino acid sequences of the CDRs of a light chain variable region are as shown in amino acid sequences at positions 24 to 34, 50 to 56, and 89 to 97 of SEQ ID NO: 21, respectively.

13. The immune-cell-targeting probe according to claim 12, wherein the heavy chain variable region of the antibody or the functional fragment thereof is as shown in SEQ ID NO: 19, and the light chain variable region is as shown in SEQ ID NO:21.

14. Use of theimmune-cell-targeting probe according to any one of claims 1 to 13 in immune cell imaging, tracing, screening and analysis.

15. The use according to claim 14, wherein the immune cell comprises, but is not limited to, lymphocytes, dendritic cells, monocyte precursors, monocytes/macrophages, neutrophils, basophils, eosinophils and mast cells.

16. A detection product, comprising the immune-cell-targeting probe according to any one of claims 1 to 13, wherein the detection product comprises, but is not limited to, a detection reagent, a detection kit or a detection chip.

17. Use of the detection product according to claim 16 in immune cell imaging, tracing, screening and analysis.

18. An immune-cell-targeting drug delivery system, wherein the immune-cell-targeting drug delivery system comprises the immune-cell-targeting probe according to any one of claims 1 to 13 and a delivery ligand.

19. The immune-cell-targeting drug delivery system according to claim 18, wherein the immune-cell-targeting probe is a combination probe formed by the binding of a targeting polypeptide and an antibody or a functional fragment thereof prepared from the targeting polypeptide as an immunogen.

20. The immune-cell-targeting drug delivery system according to claim 18, wherein the delivery ligand comprises a linker and a delivery drug.

21. An immune-cell-targeting drug delivery method, comprising the following steps:
administering first to a subject or target a targeting polypeptide connected with a pre-targeting binding handle, followed by administering a drug delivery ligand; or
mixing the targeting polypeptide connected with the pre-targeting binding handlewith the drug delivery ligand to obtain a mixture, followed by administering the mixture to the subject or target.

22. An immune-cell-targeting drug delivery method, comprising the following steps:
administering first to a subject or target an antibody or a functional fragment thereof produced by immunization with the targeting polypeptide followed by administering a drug delivery ligand; wherein the targeting polypeptide connected with the pre-targeting binding handles as described in (1) of claim 1, or
mixing the antibody or the functional fragment thereof produced by immunization with the targeting polypeptide and connected with the pre-targeting binding handle with the drug delivery ligand to obtain a mixture, followed by administering the mixture to the subject or target,
preferably wherein the antibody or a functional fragment thereof connected with the pre-targeting binding handle is as described in claim 1.

23. An immune-cell-targeting drug delivery method, comprising the following steps:
administering first to a subject or target a self-assembled mixture formed by a targeting polypeptide connected with a pre-targeting binding handle and an antibody or a functional fragment thereof produced by immunization with the targeting polypeptide and connected with the pre-targeting binding handle, followed by administering a drug delivery ligand; or
mixing the targeting polypeptide connected with the pre-targeting binding handle, the antibody or the functional fragment thereof produced by immunization with the targeting polypeptide with the drug delivery ligand to obtain a mixture, followed by administering the mixture to the subject or target.

24. The method according to any one of claims 21 to 23, wherein routes for administration comprise: intraperitoneal injection, pulmonary inhalation, direct *in vitro* administration.

25. The method according to any one of claims 21 to 23, wherein the routes for administration are the same or different when the method involves multiple administrations.

26. Use of the immune-cell-targeting drug delivery system according to any one of claims 18 to 20, utilizing immune cell recognition and pre-targeting functions, in disease diagnosis and treatment.
